(19) Europäisches Patentamt
European Patent Office
Office européen des brevets

(11) **EP 3 165 904 B1**

(12) **EUROPEAN PATENT SPECIFICATION**

(45) Date of publication and mention
of the grant of the patent:
**13.11.2019 Bulletin 2019/46**

(51) Int Cl.:
***G01N 21/61*** *(2006.01)*   ***G01N 21/3504*** *(2014.01)*
***G01N 33/00*** *(2006.01)*

(21) Application number: **15814117.6**

(22) Date of filing: **11.06.2015**

(86) International application number:
**PCT/JP2015/066872**

(87) International publication number:
**WO 2016/002466 (07.01.2016 Gazette 2016/01)**

(54) **GAS CONCENTRATION MEASUREMENT DEVICE**

GASKONZENTRATIONSMESSVORRICHTUNG

DISPOSITIF DE MESURE DE CONCENTRATION EN GAZ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **03.07.2014 JP 2014137786**

(43) Date of publication of application:
**10.05.2017 Bulletin 2017/19**

(73) Proprietor: **Murata Manufacturing Co., Ltd.
Nagaokakyo-shi, Kyoto 617-8555 (JP)**

(72) Inventors:
• **YASUDA, Masaaki**
**Nagaokakyo-shi**
**Kyoto 617-8555 (JP)**
• **TSUBONO, Masanori**
**Nagaokakyo-shi**
**Kyoto 617-8555 (JP)**
• **OGUSHI, Naoki**
**Nagaokakyo-shi**
**Kyoto 617-8555 (JP)**

(74) Representative: **Reeve, Nicholas Edward
Reddie & Grose LLP
The White Chapel Building
10 Whitechapel High Street
London E1 8QS (GB)**

(56) References cited:
EP-A1- 0 703 444    EP-A1- 2 284 904
WO-A1-2009/148134   JP-A- H08 105 833
JP-A- 2013 515 963  US-A1- 2010 027 004
US-A1- 2010 290 045 US-A1- 2013 119 254

**Description**

Technical Field

The present invention relates to an infrared-light-absorption gas concentration measurement device.

Background of the Invention

A gas concentration measurement device that uses the non-dispersive infrared (NDIR) absorption method is an example of a known concentration measurement device for measuring the concentration of a specific component contained in sample gas or the like. This type of gas concentration measurement device causes sample gas to absorb infrared light emitted from a light source, and then detects the amount of infrared light that has passed through an optical filter (band pass filter) with a detector. The concentration of the sample gas is determined on the basis of the amount of light with a specific wavelength that has been absorbed.

Japanese Unexamined Patent Application Publication No. 5-203573, for example, discloses such a gas concentration measurement device. The gas concentration measurement device disclosed in JP 5-2035731 is capable of measuring a plurality of types of sample gas by rotating a disc on which a plurality of band pass filters are arranged with intervals therebetween in a circumferential direction.

However, in the gas concentration measurement device disclosed in Japanese Unexamined Patent Application Publication No. 5-203573, the band pass filter to be used is switched by rotating the disc around a rotation axis that is parallel to the direction in which the band pass filter and the detector are arranged during the concentration measurement. The disc is relatively large because the band pass filters are arranged in the circumferential direction on the disc. Accordingly, the gas concentration measurement device is required to have a rotation region in which the disc rotates, and is therefore also large. It is difficult to install such a large gas concentration measurement device in a small space.

US2010/027004 discloses a known spectrometer with a rotatable filter assembly and a position detector rigidly attached thereto.

US2010/290045 discusses a known stereoscopic system and spectrometer employing an optical interference filter module having a plurality of bandpass regions.

US2013/119254 discusses a gas measurement module that includes an infrared source and a moveable filter member comprising at least two filter elements.

The present invention has been made in light of the above-described problem, and we have appreciated that it would be desirable to provide a gas concentration measurement device that can be reduced in size.

SUMMARY OF THE INVENTION

A gas concentration measurement device according to a first aspect of the present invention measures a gas concentration based on an absorbance of sample gas in a region between a light source that emits infrared light and a detector that detects the infrared light and is defined by the appended claims. The gas concentration measurement device includes a light source, a detector, a rotating member that is rotatable around a predetermined rotating shaft; n band pass filters, including a first band pass filter and a second band pass filter provided on the rotating member, wherein n is an integer greater than two; and a rotational driving unit that rotates the rotating member around the rotating shaft. The first band pass filter and the second band pass filter are located on a pair of planes that intersect each other, and assuming that a polygon with 2n vertices surrounding the rotation shaft is defined, the n band pass filters are located on n planes along n continuous sides of the polygon. The rotational driving unit rotates the rotating member around the rotating shaft to switch between a first state, in which the infrared light from the light source is transmitted through the first band pass filter, and a second state, in which the infrared light from the light source is transmitted through the second band pass filter; and the rotating shaft is arranged so that a direction of the rotating shaft intersects the infrared light transmitting through the first band pass filter or the second band pass filter.

In the gas concentration measurement devices according to the first aspect of the present invention, the first band pass filter is preferably a band pass filter that transmits the infrared light in an absorption band of the sample gas. In addition, the second band pass filter is preferably a band pass filter that does not transmit the infrared light in the absorption band of the sample gas but transmits the infrared light with a wavelength different from a wavelength of the infrared light transmitted by the first band pass filter.

The n band pass filters are located on n planes that intersect each other. In this case, when a crossing angle between each of the n planes and another plane that is adjacent to each of the n planes is θ [degrees], Expression (1) given below is preferably satisfied:

$$[\{(n-1)\times 180\}/n]-5 \leq \theta \leq [\{(n-1)\times 180\}/n]+5 \quad \text{Expression (1)}$$

In the gas concentration measurement devices according to the first and second aspects of the present invention, the rotational driving unit preferably includes a stepper motor.

Preferably, the gas concentration measurement devices according to the first and second aspects of the present invention further include a housing. In this case, the housing preferably contains a sample cell in which the rotating member is disposed and the rotational driving unit.

According to the present invention, a gas concentration measurement device that can be reduced in size can be provided.

BRIEF DESCRIPTION OF DRAWINGS

Fig. 1 is a top view of a gas concentration measurement device according to a first example.

Fig. 2 is a sectional view taken along line II-II in Fig. 1, illustrating a first state of a rotating member.

Fig. 3 is a sectional view taken along line II-II in Fig. 1, illustrating a second state of the rotating member.

Fig. 4 is a schematic diagram of the rotating member illustrated in Fig. 1.

Fig. 5 is a schematic diagram of a main portion of the rotating member illustrated in Fig. 4.

Fig. 6 is a schematic diagram illustrating outer side surfaces of the rotating member illustrated in Fig. 4.

Fig. 7 is a sectional view of a gas concentration measurement device according to a first embodiment, illustrating a first state of the rotating member.

Fig. 8 is a sectional view of the gas concentration measurement device according to the first embodiment, illustrating a second state of the rotating member.

DETAILED DESCRIPTION OF EXAMPLE EMBODIMENTS

An example and an embodiment of the present invention will be described in detail with reference to the drawings. In the example/embodiment described below, components that are the same or similar are denoted by the same reference numerals in the drawings, and descriptions thereof will not be repeated.

First I Example

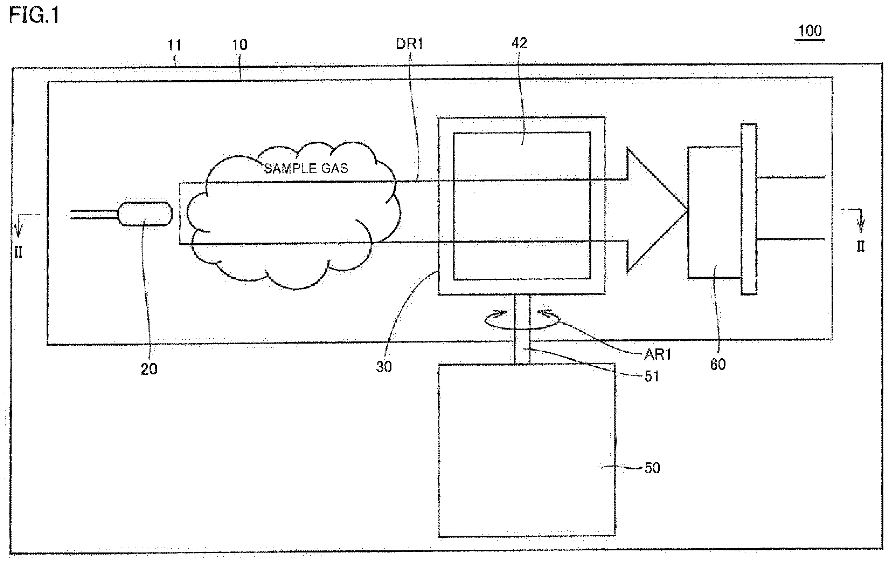

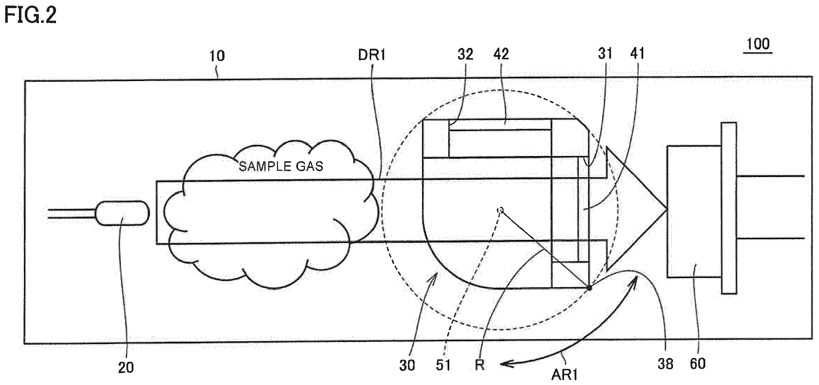

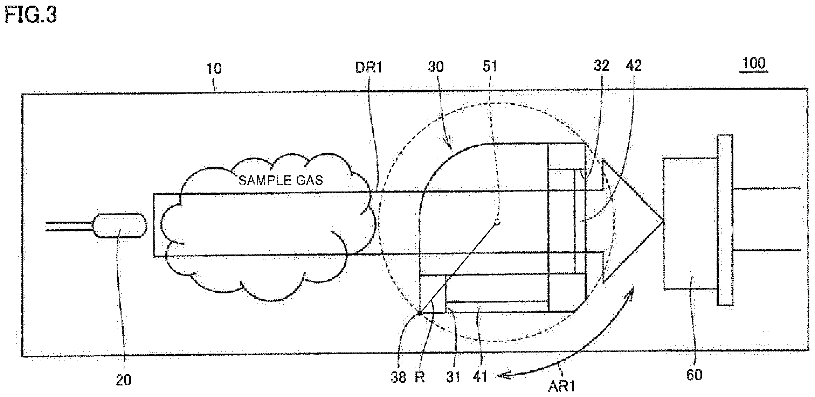

Fig. 1 is a top view of a gas concentration measurement device according to example, not covered by the present claims. Fig. 2 is a sectional view taken along line II-II in Fig. 1, illustrating a first state of a rotating member. Fig. 3 is a sectional view taken along line II-II in Fig. 1, illustrating a second state of the rotating member. A gas concentration measurement device 100 according to the present embodiment will be described with reference to Figs. 1 to 3.

As illustrated in Figs. 1 to 3, the gas concentration measurement device 100 according to the present example includes a housing 11 containing a sample cell 10 and a rotational driving unit 50. The gas concentration measurement device 100 also includes the sample cell 10, a light source 20, a rotating holder 30, a first band pass filter 41 (see Fig. 2), a second band pass filter 42, and a detector 60. The rotating holder 30 corresponds to a "rotating member" according to the present embodiment.

The gas concentration measurement device 100 measures a gas concentration in accordance with the absorbance of sample gas that flows through a space between the light source 20, which emits infrared light, and the detector 60, which includes a light-receiving portion 62 that receives the infrared light.

The sample cell 10 includes a sample-gas flow space and allows the sample gas to flow therethrough. For example, a sample-gas introduction hole (not shown) is connected to one end of the sample cell 10 (end close to the light source 20 in Fig. 1), and a sample-gas discharge hole (not shown) is connected to the other end of the sample cell 10 (end close to the detector 60 in Fig. 1). The sample gas introduced into the sample cell 10 through the sample-gas introduction hole is discharged through the sample-gas discharge hole.

The sample cell 10 contains the light source 20, the rotating holder 30, and the detector 60. The light source 20, the rotating holder 30, and the detector 60 are arranged, for example, in that order from one end of the sample cell 10. The rotational driving unit 50 is disposed outside the sample cell 10. The sample cell 10 may be either defined as a portion of the housing 11 or formed separately from the housing 11.

The light source 20 emits infrared light. The light source 20 may be, for example, a filament lamp or an LED lamp that emits wide-band infrared light including desired infrared light. Part of the infrared light emitted from the light source 20 is absorbed depending on infrared light absorption wavelength characteristics of the sample gas. The infrared light emitted from the light source 20 mainly travels in an optical axis direction (direction of arrow DR1 in the drawings) and reaches the detector 60.

The first band pass filter 41 and the second band pass filter 42 are provided on the rotating holder 30. More specifically, the first band pass filter 41 and the second band pass filter 42 are held by the rotating holder 30 in a region around a predetermined rotating shaft 51, which will be described below. The rotating holder 30 is rotated by the rotational driving unit 50 so that the first band pass filter 41 and the second band pass filter 42 are selectively disposed at a transmitting position, at which the infrared light emitted from the light source 20 is transmitted toward the detector 60.

The first band pass filter 41 is configured to transmit the infrared light in an absorption band of the sample gas to be detected. Thus, only the infrared light having a desired wavelength band reaches the detector 60. The sample gas to be detected is, for example, carbon dioxide, and the absorption band thereof is about 4.3 $\mu$m.

The second band pass filter 42 is configured not to transmit the infrared light in the absorption band of the sample gas but to transmit the infrared light with a wavelength different from that of the infrared light transmitted by the first band pass filter 41. The second band pass filter 42 transmits, for example, the infrared light in a 3.9 $\mu$m band, which is not absorbed by the sample gas.

In general, it is known that the output of the detector 60 drifts due to variations in the amount of infrared light from the light source 20 and the ambient temperature. In the present example, the second band pass filter 42 (band pass filter for reference light) and the first band pass filter 41 are switched to calculate the amount of change in the value of wavelength of the absorption band of the sample gas with respect to the value of wavelength at which the sample gas is not absorbed, so that the sensitivity can be corrected. Accordingly, the detection sensitivity of the detector 60 can be maintained constant for a long time.

The second band pass filter 42 may instead be configured to transmit the infrared light in an absorption band of another type of sample gas to be detected. In such a case, the concentrations of a plurality of types of sample gas can be measured.

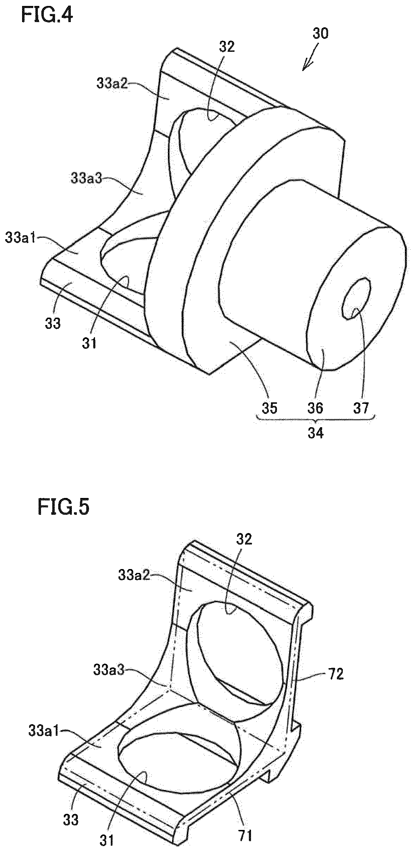

The rotating holder 30 holds the first band pass filter 41 and the second band pass filter 42 in a first through hole 31 and a second through hole 32, respectively, and is rotatable around the rotating shaft 51 (in the direction denoted by AR1 in Fig. 1). The first through hole 31 and the second through hole 32 will be described below. The rotating holder 30 holds the first band pass filter 41 and the second band pass filter 42 so that the first band pass filter 41 and the second band pass filter 42 are located on planes 71 and 72 (see Fig. 5) that intersect each other. The rotating holder 30 includes a maximum radius portion 38 at which the radius of gyration R around the rotating shaft 51 is at a maximum. The inner diameter of the sample cell 10 is set so as to be greater than twice the radius of gyration R to enable the rotation of the rotating holder 30. The rotating shaft 51 is arranged to be substantially parallel to the pair of planes 71 and 72, intersecting each other, on which the first band pass filter 41 and the second band pass filter 42 are located.

The rotational driving unit 50 is connected to the rotating holder 30 by the rotating shaft 51. The rotating shaft 51 extends through a hole portion (not shown) formed in the sample cell. Thus, the rotating shaft 51 connects the rotating holder 30, which is contained in the sample cell 10, and the rotational driving unit 50, which is disposed outside the sample cell 10.

The rotational driving unit 50 rotates the rotating holder 30 around the rotating shaft 51 to switch between the first state (see Fig. 2), in which the first band pass filter 41 is located at the above-described transmitting position, and the second state (see Fig. 3), in which the second band pass filter 42 is located at the transmitting position.

The rotational driving unit 50 rotates the rotating holder 30 around the rotating shaft 51. When switching between the first state and the second state is to be performed, the rotational driving unit 50 rotates the rotating holder 30 around the rotating shaft 51 by approximately 90 degrees. The rotating shaft 51 is arranged so as to intersect the optical axis direction of the infrared light. More specifically, the rotating shaft 51 is arranged so as to be substantially perpendicular to the optical axis direction of the infrared light. Here, "substantially perpendicular" means that the crossing angle between the rotating shaft 51 and the optical axis direction of the infrared light is in the range of 85 degrees to 95 degrees, and a case in which the above-described crossing angle varies due to assembly is included.

The rotational driving unit 50 may be, for example, a stepper motor. In the case where a stepper motor is used, the position repeatability of the rotating holder 30 can be increased when switching between the first state and the second state is performed. In addition, since the stepper motor exerts a holding torque without electricity, power consumption can be reduced.

The detector 60 may be an infrared light detector, such as a thermopile or a bolometer. The detector 60 is electrically connected to a signal processing circuit board (not shown). The detector 60 outputs an output signal to the signal processing circuit board based on the infrared light received by the light-receiving portion 62. The signal processing circuit board calculates the concentration of the sample gas based on the output signal.

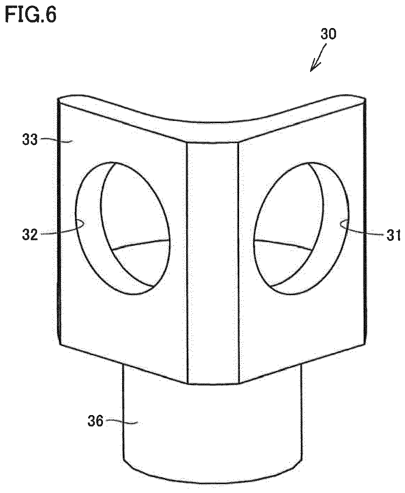

Fig. 4 is a schematic diagram of the rotating member illustrated in Fig. 1. Fig. 5 is a schematic diagram of a main portion of the rotating member illustrated in Fig. 4. Fig. 6 is a schematic diagram illustrating outer side surfaces of the rotating member illustrated in Fig. 4. The detailed shape of the rotating holder 30 according to the present embodiment will be described in detail with reference to Figs. 4 to 6.

As illustrated in Figs. 4 to 6, the rotating holder 30 includes a main portion 33 and a rotating shaft holder 34. The main portion 33 is substantially L-shaped. The main portion 33 is a portion that holds the first band pass filter 41 and the second band pass filter 42. The main portion 33 has the first through hole 31, to which the first band pass filter 41 is securely fitted, and the second through hole 32, to which the second band pass filter 42 is securely fitted.

The inner peripheral surface of the main portion 33 includes a first flat portion 33a1, a second flat portion 33a2, and a connecting portion 33a3. The second flat portion 33a2 is flat and faces the detector 60 when the second band pass filter 42 is located at the transmitting position.

The first flat portion 33a1 is flat and is substantially parallel to the axial direction of the rotating shaft 51 and the above-described optical axis direction when the second band pass filter 42 is located at the transmitting position. The first flat portion 33a1 is closer to the light source 20 than the second flat portion 33a2 is when the second band pass filter 42 is located at the transmitting position.

The connecting portion 33a3 connects the first flat portion 33a1 and the second flat portion 33a2 to each other. The connecting portion 33a3 is curved so as to approach the first flat portion 33a1 from the second flat portion 33a2 along the direction perpendicular to the axial direction of the rotating shaft 51 and the above-described optical axis direction when the second band pass filter 42 is located at the transmitting position.

The first flat portion 33a1 and the second flat portion 33a2 are not necessarily flat, and may instead be warped to some extent. The connecting portion 33a3 is not necessarily curved, and may have, for example, a pair of flat surfaces that intersect at, for example, the right angle.

In the state in which the second band pass filter 42 is located at the transmitting position, the length of the main portion 33 in the above-described optical axis direction is substantially equal to the length of the main portion 33 in the direction perpendicular to the axial direction of the rotating shaft 51 and the above-described optical axis direction.

The first band pass filter 41, which is fitted to the first through hole 31, is located on the first plane 71. The second band pass filter 42, which is fitted to the second through hole 32, is located on the second plane 72. The first plane 71 and the second plane 72 are substantially perpendicular to each other. Here, "substantially perpendicular" means that the crossing angle θ between the first plane 71 and the second plane 72 is in the range of 85 to 95 degrees. Thus, a case in which the crossing angle between the first plane 71 and the second plane 72 differs from 90 degrees due to design errors is included.

The rotating shaft holder 34 includes a side wall portion 35 and a rotating-shaft-receiving portion 36. The side wall portion 35 is, for example, fan-shaped or rectangular in plan view. The side wall portion 35 is provided at an end of the main portion 33 that is near the rotational driving unit 50.

The rotating-shaft-receiving portion 36 projects toward the rotational driving unit 50 in a direction normal to the outer principal surface of the side wall portion 35. The rotating-shaft-receiving portion 36 is cylindrical, and has a receiving hole 37 that receives the rotating shaft 51. The rotating shaft 51 is inserted into the receiving hole 37 and fixed to the receiving hole 37 so that the rotating holder 30 rotates together with the rotating shaft 51.

As described above, in the gas concentration measurement device 100 according to the present example, the rotating holder 30 holds the first band pass filter 41 and the second band pass filter 42 in such a manner that the first band pass filter 41 and the second band pass filter 42 are located on planes that intersect each other. Switching between the first state, in which the infrared light from the light source 20 is transmitted through the first band pass filter 41, and the second state, in which the infrared light from the light source 20 is transmitted through the second band pass filter 42, is performed by rotating the rotating holder 30 around the rotating shaft.

In this case, the rotating shaft is arranged so as to intersect the optical axis direction of the infrared light. Therefore, the area in which the rotating holder 30 rotates is smaller than that in the case where the rotating shaft is arranged parallel to the optical axis direction of the infrared light as in the above-described JP 5-2035731. As a result, the size of the gas concentration measurement device 100 can be reduced.

First Embodiment

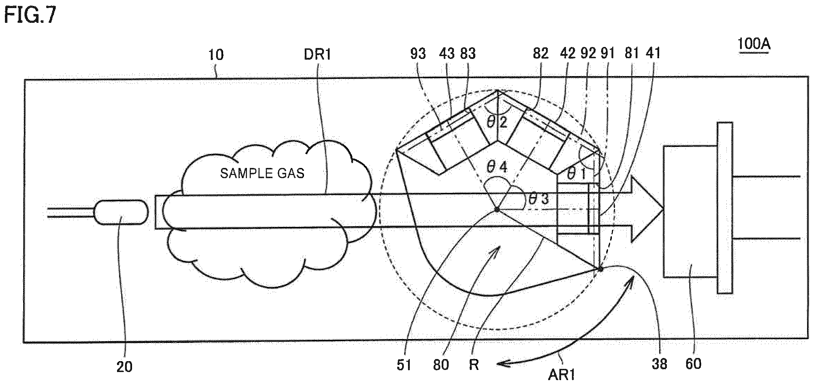

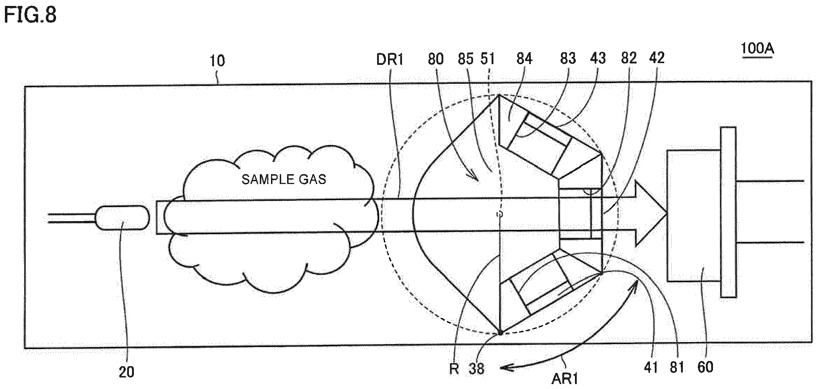

Figs. 7 and 8 are sectional views of a gas concentration measurement device according to the present embodiment, illustrating a first state and a second state, respectively, of a rotating member. The sectional views of Figs. 7 and 8 correspond to the sectional views taken along line II-II in Fig. 1. A gas concentration measurement device 100A according to the present embodiment will be described with reference to Figs. 7 and 8.

Referring to Figs. 7 and 8, in the gas concentration measurement device 100A according to the present embodiment, the structure of a rotating member 80 and the number of band pass filters differ from those in the gas concentration measurement device 100 according to the first example. The other structures, such as the structure for connecting the rotating member 80 to the rotational driving unit, of the present embodiment are substantially the same as those of the first example.

The rotating member 80 holds a first band pass filter 41, a second band pass filter 42, and a third band pass filter 43, and is rotatable around the rotating shaft 51.

The first band pass filter 41 is configured to transmit the infrared light in an absorption band of the sample gas to be detected. Thus, only the infrared light having a desired wavelength band reaches the detector 60. The sample gas to be detected is, for example, carbon dioxide, and the absorption band thereof is about 4.3 $\mu$m.

The second band pass filter 42 is configured to transmit the infrared light with a wavelength different from that of the infrared light transmitted by the first band pass filter 41. The second band pass filter 42 transmits, for example, the infrared light in a 3.9 $\mu$m band, which is not absorbed by the sample gas.

The third band pass filter 43 is configured to transmit the infrared light in an absorption band of another type of sample gas to be detected. Thus, only the infrared light having a desired wavelength band reaches the detector 60. The other type of sample gas to be detected is, for example, carbon monoxide, and the absorption band thereof is about 4.7 $\mu$m.

In the present embodiment, the second band pass filter 42 (band pass filter for reference light), the first band pass filter 41, and the third band pass filter 43 (band pass filter for enabling multiple measurements) are switched to calculate the amount of change in the value of wavelength of the absorption band of the sample gas with respect to the value of wavelength at which the sample gas is not absorbed, so that the sensitivity can be corrected. Accordingly, the detection sensitivity can be maintained constant for a long time, and the concentrations of a plurality of types of sample gas can be measured.

Although the other sample gas is carbon monoxide in the present embodiment, the other sample gas is not limited to this, and may instead be, for example, $CH_4$ or $NO_x$. Although the first band pass filter 41, the second band pass filter 42, and the third band pass filter 43 are arranged in that order, the order of arrangement is not limited to this, and may be changed.

The rotating member 80 includes a main portion 84 having a first through hole 81 to which the first band pass filter 41 is securely fitted, a second through hole 82 to which the second band pass filter 42 is securely fitted, and a third through hole 83 to which the third band pass filter 43 is securely fitted.

The first band pass filter 41, which is fitted to the first through hole 81, is located on a first plane 91. The second band pass filter 42, which is fitted to the second through hole 82, is located on a second plane 92. The third band pass filter 43, which is fitted to the third through hole 83, is located on a third plane 93.

The positional relationship between the first plane 91 and the second plane 92 is such that the crossing angle $\theta 1$ therebetween is approximately 120 degrees. The positional relationship between the second plane 92 and the third plane 93 is such that the crossing angle $\theta 2$ therebetween is approximately 120 degrees. Here, an angle that is approximately 120 degrees is an angle in the range of 115 degrees to 125 degrees. Thus, a case in which the crossing angles $\theta 1$ and $\theta 2$ vary due to design errors is included.

Thus, the peripheral shape of the main portion 84 of the rotating member 80 is preferably one-half of a regular hexagon when viewed in the rotation axis direction. The first through hole 81, the second through hole 82, and the third through hole 83 described above are arranged on the respective side surfaces of the main portion 84 having the shape of a portion of the regular hexagon when viewed in the rotation axis direction. Therefore, when viewed in the rotation axis direction, the angle $\theta 3$ between the line that connects the rotation center and the center of the first band pass filter 41 and the line that connects the rotation center and the center of the second band pass filter 42 is approximately 60 degrees, and the angle $\theta 4$ between the line that connects the rotation center and the center of the second band pass filter 42 and the line that connects the rotation center and the center of the third band pass filter 43 is approximately 60 degrees. Therefore, the band pass filter that is located at the transmitting position can be switched by rotating the rotating holder 30 by approximately 60 degrees around the rotating shaft.

In the case where switching between a plurality of band pass filters is performed, the angle by which the rotating holder 30 is rotated around the rotating shaft to switch between the band pass filters is preferably set to an integer multiple of a feed angle corresponding to a single step of a stepper motor. In the case where an ordinary motor other than a stepper motor is used, mechanical positioning may be performed by using a projection and an abutting-positioning portion, such as an abutting surface.

According to the above-described structure, also in the present embodiment, the rotating shaft is arranged so as to intersect the optical axis direction of the infrared light. Therefore, similar to the first example, the size of the gas concentration measurement device 100A can be reduced. In addition, since a plurality of band pass filters are provided, the concentrations of a plurality of types of sample gas can be measured.

The first example and first embodiments respectively describe the case in which two band pass filters are held by the rotating holder 30 and the case in which three band pass filters are held by the rotating member 80. However, the number of band pass filters held by the rotating member is not limited to this, and may instead be four or more. In this case, the peripheral shape of the main portion of the rotating member is preferably one-half of a regular polygon in which the number of sides is twice the number of band pass filters when viewed in the rotation axis direction. In this case, first and second band pass filters that are adjacent to each other are respectively located on first and second planes that are different from each other, and the angle between the first and second planes is substantially equal to the interior angle of the regular polygon.

More specifically, in the case where n band pass filters are provided (n is an integer greater than 2), the n band pass filters are arranged on n planes that intersect each other. In this case, when the crossing angle between each of the n planes and another plane that is adjacent to each of the n planes is θ [degrees], Expression (1) is preferably satisfied. Expression (1) shows that the crossing angle θ varies in the range of ±5 degrees due to design errors.

$$[\{(n-1)\times 180\}/n]-5 \leq \theta \leq [\{(n-1)\times 180\}/n]+5 \quad \text{Expression (1)}.$$

Also when the above-described structure is employed, the rotating shaft is arranged so as to intersect the optical axis direction of the infrared light. Therefore, similar to the above-described case, the size of the gas concentration measurement device can be reduced. In addition, the concentrations of a plurality of types of sample gas can be measured.

Although embodiments of the present invention have been described, the embodiment disclosed herein are illustrative and not restrictive in all points. The scope of the present invention is to be defined by the scope of the claims, and includes equivalents to the scope of the claims and all changes within the scope of the claims.

Reference Signs List

10 sample cell, 20 light source, 30 rotating holder, 31 first through hole, 32 second through hole, 33 main portion, 33a1 first flat portion, 33a2 second flat portion, 33a3 connecting portion, 34 rotating shaft holder, 35 side wall portion, 36 rotating-shaft-receiving portion, 37 receiving hole, 38 maximum radius portion, 41 first band pass filter, 42 second band pass filter, 43 third band pass filter, 50 rotational driving unit, 51 rotating shaft, 60 detector, 62 light-receiving portion, 71 first plane, 72 second plane, 80 rotating member, 81 first through hole, 82 second through hole, 83 third through hole, 84 main portion, 91 first plane, 92 second plane, 93 third plane, 100 and 100A gas concentration measurement device.

**Claims**

1. A gas concentration measurement device (100) that measures a gas concentration based on an absorbance of sample gas in a region between a light source (20) that emits infrared light and a detector (60) that detects the infrared light, the gas concentration measurement device comprising:

   a light source (20);
   a detector (60);
   a rotating member (30) that is rotatable around a predetermined rotating shaft (51);
   n band pass filters, including a first band pass filter (41) and a second band pass filter (42) provided on the rotating member (30), wherein n is an integer greater than two; and
   a rotational driving unit (50) that rotates the rotating member (30) around the rotating shaft (51), wherein the first band pass filter (41) and the second band pass filter (42) are located on a pair of planes that intersect each other, and
   assuming that a polygon with 2n vertices surrounding the rotating shaft is defined, the n band pass filters are located on n planes along n adjacent sides of the polygon, wherein the rotational driving unit (50) rotates the rotating member (30) around the rotating shaft (51) to switch between a first state, in which the infrared light from the light source (20) is transmitted through the first band pass filter (41), and a second state, in which the infrared light from the light source (20) is transmitted through the second band pass filter (42); and the rotating shaft is arranged so that a direction of the rotating shaft intersects with the direction of the infrared light transmitting through the first band pass filter or the second band pass filter.

2. The gas concentration measurement device according to claim 1 , wherein the first band pass filter (41) is a band pass filter that transmits the infrared light in an absorption band of the sample gas, and
   wherein the second band pass filter (42) is a band pass filter that does not transmit the infrared light in the absorption band of the sample gas but transmits the infrared light with a wavelength different from a wavelength of the infrared light transmitted by the first band pass filter.

3. The gas concentration measurement device according to claims 1 or 2, wherein the n band pass filters are located on n planes that intersect each other, and when a crossing angle between each of the n planes and another plane that is adjacent to each of the n planes is θ [degrees], the following expression is satisfied:

$$[\{(n-1)\times180\}/n]-5 \leq \theta \leq [\{(n-1)\times180\}/n]+5 \ .$$

**4.** The gas concentration measurement device according to any one of claims 1 to 3, wherein the rotational driving unit includes a stepper motor (50).

**5.** The gas concentration measurement device according to any one of claims 1 to 4, further comprising a housing, wherein the housing contains a sample cell in which the rotating member is disposed and the rotational driving unit.

**Patentansprüche**

**1.** Gaskonzentrationsmessvorrichtung (100), die eine Gaskonzentration auf der Basis einer Absorbanz von Probengas in einer Region zwischen einer Lichtquelle (20), die Infrarotlicht emittiert, und einem Detektor (60) misst, der das Infrarotlicht erkennt, wobei die Gaskonzentrationsmessvorrichtung Folgendes umfasst:

eine Lichtquelle (20);
einen Detektor (60);
ein rotierendes Element (30), das um eine vorbestimmte rotierende Welle (51) gedreht werden kann;
n Bandpassfilter, einschließlich einem ersten Bandpassfilter (41) und einem zweiten Bandpassfilter (42), vorgesehen auf dem rotierenden Element (30), wobei n eine ganze Zahl größer als zwei ist; und
eine Drehantriebseinheit (50), die das rotierende Element (30) um die Drehwelle (51) dreht, wobei sich das erste Bandpassfilter (41) und das zweite Bandpassfilter (42) auf einem Paar Ebenen befinden, die einander schneiden, und
unter der Annahme, dass ein Polygon mit 2n Eckpunkten um die Drehwelle definiert wird, befinden sich die n Bandpassfilter auf n Ebenen entlang n benachbarten Seiten des Polygons,
wobei die Drehantriebseinheit (50) das rotierende Element (30) um die Drehwelle (51) dreht, um zwischen einem ersten Zustand, in dem das Infrarotlicht von der Lichtquelle (20) durch das erste Bandpassfilter (41) gelassen wird, und einem zweiten Zustand dreht, in dem das Infrarotlicht von der Lichtquelle (20) durch das zweite Bandpassfilter (42) gelassen wird; und die Drehwelle so angeordnet ist, dass eine Richtung der Drehwelle die Richtung des durch das erste Bandpassfilter oder das zweite Bandpassfilter gelassenen Infrarotlichts schneidet.

**2.** Gaskonzentrationsmessvorrichtung nach Anspruch 1, wobei das erste Bandpassfilter (41) ein Bandpassfilter ist, das das Infrarotlicht in einem Absorptionsband des Probengases durchlässt, und
wobei das zweite Bandpassfilter (42) ein Bandpassfilter ist, das das Infrarotlicht im Absorptionsband des Probengases nicht durchlässt, sondern das Infrarotlicht mit einer Wellenlänge durchlässt, die sich von einer Wellenlänge des vom ersten Bandpassfilter durchgelassenen Infrarotlichts unterscheidet.

**3.** Gaskonzentrationsmessvorrichtung nach Anspruch 1 oder 2, wobei sich die n Bandpassfilter auf n Ebenen befinden, die einander schneiden, und wenn ein Kreuzungswinkel zwischen jeder der n Ebenen und einer anderen Ebene, die sich neben jeder der n Ebenen befindet, θ [Grad] ist, dann wird der folgende Ausdruck erfüllt:

$$[\{(n-1)x180\}/n]-5 \leq \theta \leq [\{(n-1)x180\}/n]+5.$$

**4.** Gaskonzentrationsmessvorrichtung nach einem der Ansprüche 1 bis 3, wobei die Drehantriebseinheit einen Schrittmotor (50) beinhaltet.

**5.** Gaskonzentrationsmessvorrichtung nach einem der Ansprüche 1 bis 4, die ferner ein Gehäuse umfasst, wobei das Gehäuse eine Probenzelle enthält, in der das rotierende Element und die Drehantriebseinheit angeordnet sind.

**Revendications**

**1.** Dispositif de mesure de concentration en gaz (5) qui mesure une concentration en gaz basé sur une absorbance d'un échantillon de gaz dans une région entre une source lumineuse (20) qui émet une lumière infrarouge et un détecteur (60) qui détecte la lumière infrarouge, le dispositif de mesure de concentration en gaz comprenant :

une source lumineuse (20) ;

un détecteur (60) ;

un élément rotatif (30) qui peut tourner autour d'un arbre de rotation prédéterminé (51) ;

n filtres passe-bande, comportant un premier filtre passe-bande (41) et un second filtre passe-bande (42) fournis sur l'élément rotatif (30), dans lequel n est un entier supérieur à deux ; et

une unité d'entraînement rotationnel (50) qui fait tourner l'élément rotatif (30) autour de l'arbre rotatif (51), dans lequel le premier filtre passe-bande (41) et le second filtre passe-bande (42) sont situés sur une paire de plans qui s'interceptent l'un l'autre, et

en supposant qu'un polygone à 2n sommets entourant l'arbre de rotation est défini, les n filtres passe-bande sont situés sur n plans le long de n côtés adjacents du polygone,

dans lequel l'unité d'entraînement rotationnel (50) fait tourner l'élément rotatif (30) autour de l'arbre rotatif (51) pour commuter entre un premier état, dans lequel la lumière infrarouge provenant de la source lumineuse (20) est transmise à travers le premier filtre passe-bande (41), et un second état, dans lequel la lumière infrarouge provenant de la source lumineuse (20) est transmise à travers le second filtre passe-bande (42) ; et l'arbre rotatif est agencé de telle sorte qu'un sens de l'arbre rotatif intersecte le sens de la lumière infrarouge transmise à travers le premier filtre passe-bande ou le second filtre passe-bande.

2. Dispositif de mesure de concentration en gaz selon la revendication 1, dans lequel le premier filtre passe-bande (41) est un filtre passe-bande qui transmet la lumière infrarouge dans une bande d'absorption de l'échantillon de gaz, et

dans lequel le second filtre passe-bande (42) est un filtre passe-bande qui ne transmet pas la lumière infrarouge dans la bande d'absorption de l'échantillon de gaz mais transmet la lumière infrarouge avec une longueur d'onde différente d'une longueur d'onde de la lumière transmise par le premier filtre passe-bande.

3. Dispositif de mesure de concentration en gaz selon les revendications 1 ou 2, dans lequel les n filtres passe-bande sont situés sur n plans qui s'intersectent l'un l'autre, et quand un angle de croisement entre chacun des n plans et un autre plan qui est adjacent à chacun des n plans est $\theta$ [degrés], l'expression suivante est satisfaite :

$$[\{(n-1)x180\}/n]-5 \leq \theta \leq [\{(n-1)x180\}/n]+5.$$

4. Dispositif de mesure de concentration en gaz selon l'une quelconque des revendications 1 à 3, dans lequel l'unité d'entraînement rotationnel comporte un moteur pas à pas (50).

5. Dispositif de mesure de concentration en gaz selon l'une quelconque des revendications 1 à 4, comprenant en outre un boîtier,

dans lequel le boîtier contient une cellule à échantillon dans laquelle l'élément rotatif est disposé et l'unité d'entraînement rotationnel.

FIG.1

SAMPLE GAS

11  10  DR1  42

II

20  30  AR1  51

50  60

FIG.2

SAMPLE GAS

EP 3 165 904 B1

FIG.3

FIG.4

FIG.5

FIG.6

FIG.7

EP 3 165 904 B1

FIG.8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5203573 A
- JP 52035731 B
- US 2010027004 A
- US 2010290045 A
- US 2013119254 A